# EUROPEAN PATENT SPECIFICATION

(11) **EP 3 027 176 B1**
(45) Date of publication and mention of the grant of the patent: **23.08.2017**
(21) Application number: 14753025.7
(22) Date of filing: 25.07.2014
(51) Int. Cl.: A61K 9/24, A61K 9/28, A61K 31/4365, A61K 31/60, A61K 9/20

(54) **PHARMACEUTICAL TABLET COMPRISING ACETYLSALICYLIC ACID AND CLOPIDOGREL**
PHARMAZEUTISCHE TABLETTE ENTHALTEN ACETYLSALICYLSÄURE UND CLOPIDROGEL
COMPRIMÉ PHARMACEUTIQUE COMPRENANT DE L'ACIDE ACÉTYLSALICYLIQUE ET DU CLOPIDOGREL

(30) Priority: 02.08.2013 WO PCT/FR2013/051874
(43) Date of publication of application: 08.06.2016
(73) Proprietor: SANOFI, 75008 Paris (FR)
(72) Inventor: BRENNE, Jean-François, F-75008 Paris (FR); CALLIGARIS, Frédérique, F-75008 Paris (FR); DASTE, Georges, F-75008 Paris (FR); EDELINE-BERLEMONT, Julie, F-75008 Paris (FR); FONTAINE, Nicolas, F-75008 Paris (FR)
(74) Representative: Catillon, Marie
(86) International application number: PCT/EP2014/066110
(87) International publication number: WO 2015/014766

(56) References cited:
- WO-A1-97/29753
- WO-A1-2011/110989
- FR-A1- 2 792 836

## Description

The present invention relates to a pharmaceutical tablet containing a combination of active ingredients with platelet aggregation-inhibiting activity, consisting of acetylsalicylic acid, or a pharmaceutically acceptable salt thereof, and of clopidogrel or a pharmaceutically acceptable salt thereof.

The therapeutic advantage of the combination of acetylsalicylic acid, also known as aspirin, and of clopidogrel is, inter alia, described in international applications WO 97/29753 and WO 00/66130.

WO 00/66130 describes more particularly a unit pharmaceutical form, such as a tablet, a gel capsule or a single-dose packet, comprising acetylsalicylic acid and clopidogrel.

However, none of the pharmaceutical forms disclosed in that application makes it possible to minimise the possible gastric lesions that can be caused by the ingestion of aspirin.

It has proved to be useful and advantageous to have an orally administrable unit pharmaceutical form which contains these two active ingredients and which protects the gastric mucosa. Indeed, it turns out to be the case that a particular sensitivity to aspirin is exhibited by a part of the population, for whom it is preferable for aspirin to be sold in a form that is non-aggressive to the gastric mucosa.

The work of the inventors has made it possible to produce such a pharmaceutical form.

The invention therefore relates to a pharmaceutical tablet comprising a core and an external layer, the core comprising acetylsalicylic acid or a pharmaceutically acceptable salt thereof and the external layer comprising clopidogrel or a pharmaceutically acceptable salt thereof, the core being separated from the external layer by a three-layer coating composed of a gastro-resistant layer inserted between two isolating layers, the gastro-resistant layer comprising at least one gastro-resistant polymer and at least 40% by weight of talc, relative to the weight of gastro-resistant polymer.

The tablet according to the invention has the same therapeutic effects as the tablet described in application WO 00/66130, while at the same time having aspirin-gastroresistance properties for protecting the population which exhibits a particular sensitivity to aspirin. Unexpectedly, the tablet according to the invention also exhibits better stability. In particular, as is detailed in Example 3, the tablet according to the invention can be stored for 36 months, without applying particular storage conditions.

It will be noted that, in the context of the present application, and unless otherwise stipulated, the ranges of values indicated are understood to be limits included.

Among the various possible pharmaceutical forms, the formulation in tablet form has many advantages. The term "tablet" is understood to mean a pharmaceutical form of solid consistency, obtained by compression of particles such as particles of powders and/or granules, and containing a dose of medicament.

In particular, a tablet has the following advantages:
- it is very resistant and enables easy manipulation,
- the dosage of the active ingredients in a tablet is very precise,
- unlike powder, taking a tablet does not require prior dispersion in water,
- the tablet form allows the introduction of a large amount of active ingredients in a restricted volume, and, finally,
- the industrial production of tablets can be carried out at high rates.

In the tablets according to the invention, the acetylsalicylic acid or a pharmaceutically acceptable salt thereof is preferably present in its acid form and more preferentially starched, while the clopidogrel or a pharmaceutically acceptable salt thereof is preferably present in the form of clopidogrel hydrogen sulfate of formula I below: 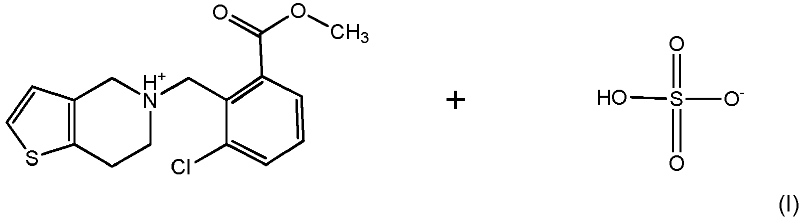 and more preferentially in the polymorphic form II. The form II of clopidogrel hydrogen sulfate and a process for obtaining this form are more particularly described in application WO 99/65915.

The term "coating" is intended to mean the application of a layer to the whole of the surface of the core. In particular, for the three-layer coating according to the invention, each layer making up this coating is applied to the whole of the surface of the core, said core optionally bearing a first layer (internal isolating layer) and optionally a second layer (gastro-resistant layer).

The term "isolating layer" is intended to mean a layer which makes it possible to avoid direct contact between two components of the tablet. More particularly, the tablet according to the invention comprises two isolating players, the internal isolating layer making it possible to avoid direct contact between the core and the gastro-resistant layer, and the external isolating layer making it possible to avoid direct contact between the gastro-resistant layer and the external layer. Even more particularly, the internal isolating layer makes it possible to protect the gastro-resistant layer from the acid ingredients contained in the core (acetylsalicylic acid or a pharmaceutically acceptable salt thereof), and the external isolating layer makes it possible to protect the gastro-resistant layer from the acid ingredients contained in the external layer (clopidogrel or a pharmaceutically acceptable salt thereof).

The two isolating layers according to the invention comprise a film-forming agent which forms a barrier between two components having different pHs. Such film-forming agents are known to those skilled in the art and mention may be made, by way of example, of hydroxypropylmethylcellulose, hydroxypropylcellulose, polymethacrylates, polyvinyl alcohol, polyvinylpyrrolidone (povidone), vinyl acetate-vinylpyrrolidone (copovidone) copolymer and shellac resin. Preferably, the film-forming agent is hydroxypropylmethylcellulose, hydroxypropylcellulose and/or polyvinyl alcohol, more preferentially hydroxypropylmethylcellulose, and even more preferentially low-viscosity hydroxypropylmethylcellulose.

Advantageously, the isolating layer comprises a film-forming agent in combination with a polyethylene glycol, preferably PEG 6000. The addition of polyethylene glycol is capable of improving the quality of the isolating layer. As will be subsequently detailed, the tablet according to the invention is obtained via a double compression, which step is capable of damaging the three-layer coating and/or causing the tablet to lose its property or properties of gastroresistance in the stomach (at the stomach pH) and/or of dissolution in the intestines (at the intestinal pH).

In particular, the isolating layer is devoid of titanium dioxide.

It will be noted that the internal and external isolating layers may have an identical or different chemical composition and/or thickness.

The term "gastro-resistant layer" is therefore intended to mean a layer comprising at least one gastro-resistant polymer. Gastro-resistant polymers are well known to those skilled in the art. These polymers dissolve only at a pH above 5.5 and make it possible to protect the gastric environment from the ingredient or the composition that they coat, namely, in the present case, the acetylsalicylic acid or a pharmaceutically acceptable salt thereof contained in the core. This ingredient or composition is finally released only in the duodenum.

Advantageously, the gastro-resistant polymer is a copolymer of methacrylic acid and of ethyl acrylate. Such a copolymer is sold under the brand Eudragit® (Evonik industries), in the form of an aqueous dispersion under the reference L 30 D-55, or in dry form under the reference L 100-55.

More preferentially, for simplicity of implementation, Eudragit L 30 D-55 is used. Indeed, the suspension of copolymer is easily sprayed onto the core, and then dried.

Eudragit L 30 D-55 is an aqueous dispersion at 30% by weight of dry copolymer of methacrylic acid and of ethyl acrylate having an average molecular weight of 250 000 and a carboxyl group/ester group ratio approximately equal to 1:1, also comprising 0.7% by weight of sodium lauryl sulfate and 2.3% by weight of polysorbate 80 (also known as polyoxyethylene (20) monooleate or polyoxyethylene sorbitan monooleate 80), the sodium lauryl sulfate and polysorbate 80 content being given relative to the weight of solids of the copolymer.

The gastro-resistant layer also comprises at least 40% by weight of talc, relative to the weight of gastro-resistant polymer. More particularly, the percentage by weight of talc is given relative to the dry weight of gastro-resistant polymer.

Preferably, the gastro-resistant layer comprises from 40% to 150%, more preferentially from 45% to 105% by weight of talc, relative to the dry weight of gastro-resistant polymer. These talc contents make it possible to obtain a tablet which has an intact gastro-resistant layer. The term "intact" is intended to mean a gastro-resistant layer which has not been damaged by the double compression step, which does not show any cracks and which is resistant to the disintegration test in acidic medium (at a pH=1.2), such as the one more thoroughly described in Example 2.

The gastro-resistant layer can also advantageously comprise a plasticiser. Preferably, the plasticiser these triethyl citrate or triethylacetyl citrate, more preferentially triethyl citrate.

A preferred composition of the gastro-resistant layer, after drying, according to the invention is indicated hereinafter:
- 25%-60% by dry weight of copolymer of methacrylic acid and of ethyl acrylate,
- 25%-60% by weight of talc,
- 8%-15% by weight of triethyl citrate,
these percentages by weight being given on the basis of the total dry weight of the gastro-resistant layer.

Indicated hereinafter, by way of example, is a composition of the gastro-resistant layer, after drying, when it comprises approximately 50% by weight of talc, relative to the dry weight of gastro-resistant polymer:
- 57%-61% by dry weight of gastro-resistant polymer, for example of copolymer of methacrylic acid and of ethyl acrylate,
- 28%-31 % by weight of talc,
- 8%-15% by weight of plasticiser, for example of triethyl citrate,
the percentages by weight being given on the basis of the total dry weight of the gastro-resistant layer.

From the point of view of its structure, the tablet is a cored tablet. The core is centred in the tablet and has a shape identical or similar to that of the tablet. These parameters can make it possible to contribute towards preserving the integrity of the three-layer coating during the double compression carried out in order to prepare the tablet.

The tablet according to the invention has a volume of less than 500 mm³, preferably less than 450 mm³ and more preferentially less than 420 mm³.

It will be noted that, by virtue of the invention, it is possible to obtain a tablet of small size. Indeed, an easy solution for preserving the integrity of the gastro-resistant layer could have been to increase the size of the tablet in order to minimise the impact of the double compression on the gastro-resistant layer. However, such a solution would have resulted in a reduction in the patient's comfort when ingesting the tablet, owing to its large size.

The term "tablet" is intended to mean not only uncoated tablets, but also film coated tablets. The term "uncoated" is intended to mean the tablet devoid of any film coating applied to the external layer.

Advantageously, the uncoated tablet is approximately 390 mm³.

According to one embodiment, the uncoated tablet has a cylindrical general shape, with a convex upper and lower face, a radius of less than 5.7 mm, preferably between 5.5 and 5.6 mm, and a maximum thickness of 5.9 mm, preferably less than 5.8 mm.

Once the film coating has been applied to the external layer of the tablet, the volume of the film-coated tablet is approximately 410 to 420 mm³. The film-coated tablet still has a radius of less than 5.7 mm, preferably between 5.55 and 5.65 mm, and a maximum thickness of 6.1 mm, preferably less than 6.0 mm.

Such geometry of the tablet allows said tablet to be easily ingested by the patient.

Regarding the weight, the uncoated tablet has a weight of between 455 and 570 mg, preferentially between 485-540 mg, and, once the film coating has been applied to the external layer, the weight of the film-coated tablet is approximately 480-600 mg, more preferentially 510-570 mg.

Advantageously, the gastro-resistant layer represents, after drying, at least 20% by weight of the total weight of the core. Preferably, it represents from 20% to 28% by weight and more preferentially from 22% to 26% by weight of the total weight of the core.

The tablet according to the invention is capable of releasing in vitro:
- at least 70%, preferentially 75%, more preferentially 80% and even more preferentially 85% by weight of clopidogrel or the pharmaceutically acceptable salt thereof in a first acidic dissolution medium at a pH of approximately or equal to 1.2 with a type 1 dissolution apparatus in accordance with European pharmacopoeia 2.9.3 "Dissolution test for solid dosage forms", over the course of 30 min while at the same time releasing at most 10% by weight of acetylsalicylic acid or the pharmaceutically acceptable salt thereof, over the course of 120 min, then
- at least 65%, preferentially 70%, more preferentially 75% and even more preferentially 80% by weight of acetylsalicylic acid or the pharmaceutically acceptable salt thereof in a second dissolution medium of a buffered solution at a pH of approximately or equal to 6.8 with a type 1 dissolution apparatus in accordance with European pharmacopoeia 2.9.3 "Dissolution test for solid dosage forms", over the course of 90 min.

Alternatively, the tablet according to the invention is capable of releasing in vitro:
- at least 70%, preferentially 75%, more preferentially 80% and even more preferentially 85% by weight of clopidogrel or the pharmaceutically acceptable salt thereof in a first acidic dissolution medium at a pH of approximately or equal to 1.2 with a type 1 dissolution apparatus in accordance with United States pharmacopoeia (U.S.P.) 711 "Dissolution", over the course of 30 minutes, while at the same time releasing at most 10% by weight of acetylsalicylic acid or the pharmaceutically acceptable salt thereof, over the course of 120 min, then
- at least 65%, preferentially 70%, more preferentially 75% and even more preferentially 80% by weight of acetylsalicylic acid or the pharmaceutically acceptable salt thereof in a second dissolution medium of buffered solution at a pH of approximately or equal to 6.8 with a type 1 dissolution apparatus in accordance with United States pharmacopoeia (U.S.P.) 711 "Dissolution", over the course of 90 min.

Alternatively, the tablet according to the invention is capable of releasing in vitro:
- at least 70%, preferentially 75%, more preferentially 80% and even more preferentially 85% by weight of clopidogrel or the pharmaceutically acceptable salt thereof in a first acidic dissolution medium at a pH of approximately or equal to 1.2 with a type 1 dissolution apparatus in accordance with Japanese pharmacopoeia 6.10 "Dissolution test", over the course of 30 min while at the same time releasing at most 10% by weight of acetylsalicylic acid or the pharmaceutically acceptable salt thereof, over the course of 120 min, then
- at least 65%, preferentially 70%, more preferentially 75% and even more preferentially 80% by weight of acetylsalicylic acid or the pharmaceutically acceptable salt thereof in a second dissolution medium of buffered solution at a pH of approximately or equal to 6.8 with a type 1 dissolution apparatus in accordance with Japanese pharmacopoeia 6.10 "Dissolution test", over the course of 90 min.

In the present application, any reference to a country's pharmacopoeia is understood to be a reference to the pharmacopoeia in force in the country concerned at the date of filing of the present application.

From a practical point of view, the two dissolution tests indicated above are carried out successively with the same uncoated tablet. In particular, the tablet which has undergone the first dissolution test carried out in the first dissolution medium at a pH of approximately or equal to 1.2 is recovered after 120 min and is immersed in the second dissolution medium at a pH of approximately or equal to 6.8 in order to carry out the second dissolution test.

The assays of clopidogrel and of acetylsalicylic acid dosages in the various dissolution media are carried out by liquid-phase chromatography, for example according to a methodology in accordance with European pharmacopoeia 2.2.29 "Liquid chromatography" or in accordance with United States pharmacopoeia (U.S.P.) 621 "Chromatography" or in accordance with Japanese pharmacopoeia 2.01 "Liquid Chromatography".

More particularly, the tablet according to the invention exhibits dissolution profiles similar to that of a Plavix® 75 mg tablet and that of a 100 mg gastro-resistant aspirin tablet, which are sold in Japan. More particularly, the dissolution profile of the clopidogrel contained in the tablet according to the invention meets the specifications indicated in the monograph of clopidogrel tablets of the United States pharmacopoeia (USP Monographs: Clopidogrel tablets).

In the tablet according to the invention the clopidogrel and the acetylsalicylic acid are present in a clopidogrel/acetylsalicylic acid molar ratio of between 0.35 and 0.45.

Preferably, the tablet contains 75 mg of clopidogrel (i.e. 97.880 mg of clopidogrel hydrogen sulfate) and 100 mg of acetylsalicylic acid (i.e. 111.111 mg of starched aspirin).

According to one preferred embodiment, the core has a formulation which enables its direct compression. Advantageously, the core comprises starched aspirin, a flow agent (for example, anhydrous colloidal silica), a filler (example, microcrystalline cellulose) and a lubricant (for example, stearic acid). The addition of the filler and more particularly of microcrystalline cellulose to the core makes it possible to improve the compressibility of the core with a view to the subsequent three-layer coating and double compression steps.

Likewise, the external layer of clopidogrel has a formulation suitable for the forming thereof by compression. Advantageously, it comprises clopidogrel hydrogen sulfate, at least one filler (for example, anhydrous lactose, pregelatinised starch, preferably partially pregelatinised starch, and/or microcrystalline cellulose), a disintegrating agent (for example, hydroxypropylcellulose, preferably weakly substituted hydroxypropylcellulose), a binder (for example, PEG in powder form), an antioxidant (for example, alpha-tocopherol), and at least one lubricant (for example, caster oil and/or a sucrose ester, preferably fatty acid sucrose esters).

The tablet according to the invention may also comprise a film coating. Advantageously, the film coating is composed of four successive layers:
- a first attachment layer,
- a second layer, a function of which is to protect the clopidogrel from light,
- a third layer for obtaining a smooth surface appearance and which is capable of receiving optional impressions, and
- a fourth layer aimed at promoting the sliding of the tablet during manipulation thereof.

Such a film coating is particularly suitable when it is desired to obtain a tablet with a pleasant aesthetic appearance.

The invention also relates to a process for manufacturing a tablet according to the invention, said process comprising the following steps:
(i) preparing a core comprising acetylsalicylic acid or a pharmaceutically acceptable salt thereof,
(ii) coating the core, so as to obtain a coated core, with a three-layer coating composed of a gastro-resistant layer inserted between two isolated layers,
(iii) compression-coating the coated core obtained in step (ii) with the powder comprising the clopidogrel or a pharmaceutically acceptable salt thereof, so as to obtain a tablet.

Advantageously, the core is prepared by direct compression of the ingredients of which it is composed. These ingredients are more particularly detailed above. Advantageously, the compression of the core is carried out at a compressive force ranging from 10 to 25 kN, preferably from 14 to 20 kN.

Likewise, the composition of the layers of the three-layer coating is detailed above.

The three-layer coating in step (ii) is carried out by means of three successive coating steps. Each coating operation is carried out by preparing an aqueous solution or dispersion of the mixture of ingredients making up each of the layers (internal isolating layer, gastro-resistant layer, external isolating layer) and spraying said solution or dispersion under a hot air stream in order to allow the water contained in the aqueous solution or dispersion to evaporate. The deposition of a dry film on the cores is thus obtained, said cores being optionally coated with a first and optionally with a second layer. A pause allowing the deposited layer to dry is carried out between each layer.

Advantageously, the core bearing a first isolating layer (internal isolating layer) is coated with an amount of gastro-resistant layer representing at least 20% by weight of the total weight of the core, the weights being given after drying. Preferably, the amount of gastro-resistant layer represents from 20% to 28% by weight and more preferentially from 22% to 26% by weight of the total weight of the core.

Step (iii) is aimed at coating the core with clopidogrel powder. The clopidogrel powder is obtained by mixing clopidogrel granules (obtained via the hot melt process) with pharmaceutically acceptable excipients.

The compression performed in step (iii) in order to coat the core is in fact a double compression step. The latter is in fact carried out in two substeps:
- an intermediate compression of the core coated with a first part of the clopidogrel powder in order to obtain a pre-tablet, then
- a final compression of the pre-tablet with the other part of the clopidogrel powder.

Advantageously, the ratio of the weight of the first part of the clopidogrel powder to the weight of the other part (or complementary part) of the clopidogrel powder is between 1.05 and 1.19. It appears that this parameter makes it possible to contribute to not impairing the gastroresistance of the core. For example, for an uncoated tablet of approximately 510 to 515 mg, the intermediate compression is carried out with approximately 185 (+/-5) mg of clopidogrel powder, and approximately 165 (+/-5) mg of clopidogrel powder is then added for the final compression.

It will be noted that the compressive force applied during the intermediate compression is less than the compressive force applied during the final compression.

Typically, the compressive force during the intermediate compression is included in the range of from 0.8 to 1.2 kN, while the compressive force during the final compression is included in the range from 19 to 24 kN.

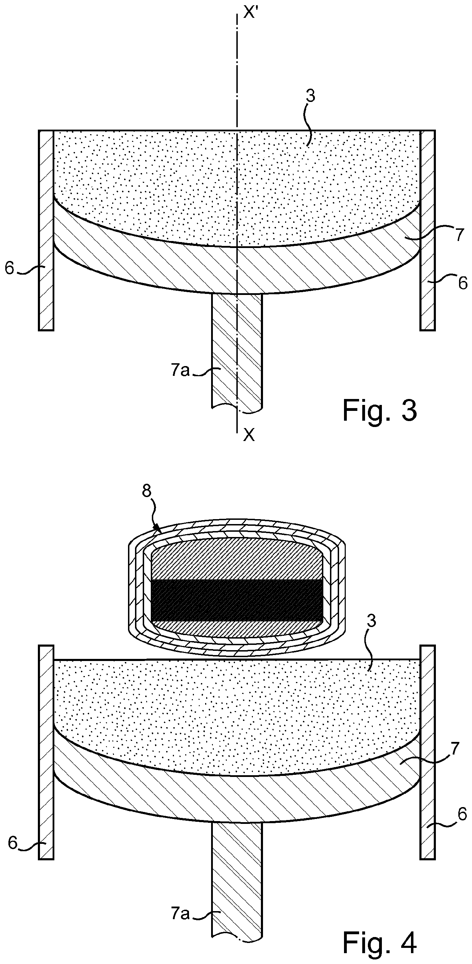

The double compression is more thoroughly detailed in Example 1 "Preparation of the tablet" with reference to Figures 3 to 7. This double compression technology is also known as "tablet in tablet" or "cored tablet".

Preferably, this double compression is carried out using a double-core rotary tableting machine.

Advantageously, the ratio of the weight of the external layer to the weight of the core is included in a range from 2.5 to 3.2.

According to one preferential embodiment, the manufacturing process also comprises a film coating step. Advantageously, the film coating comprises four successive layers, the first three layers (attachment layer, protective layer and surface appearance layer) being deposited by spraying, and the final layer (sliding layer) by powdering. This film coating detailed more thoroughly in Example 1 which follows.

The invention is also directed towards the tablets according to the invention, for use in the treatment of a pathological condition induced by platelet aggregation, including stable or unstable angina, cardiovascular and cerebrovascular system disorders, for instance thromboembolic disorders associated with atherosclerosis and with diabetes, such as unstable angina, stroke, post-angioplasty restenosis, endarterectomy or the insertion of metal endovascular prostheses, or thromboembolic disorders associated with the post-thrombolysis re-thrombosis, with infarction, with dementia of ischaemic origin, with peripheral arterial diseases, with haemodialysis, with atrial fibrillation, or else during the use of vascular prostheses, of aortocoronary bypasses, or else during radiotherapy in order to reduce the side effects thereof.

The invention will be understood more clearly in the light of the examples which follow, which are given by way of illustration with reference to the figures, which represent respectively:
- Figure 1 is a diagrammatic sectional view of a tablet 1 according to the invention,
- Figure 2 is an enlarged view of the detail A of Figure 1 representing the three-layer coating 4 separating the core 2 from the external layer 3,
- Figure 3 is a diagrammatic partial sectional view of a compression unit of a tablet-producing machine, after a first step of filling with a part of the powder intended to form the external layer 3,
- Figure 4 is a view analogous to that of Figure 3, after positioning of a coated core 8,
- Figure 5 is a view analogous to those of Figures 3 and 4, during an intermediate compression step,
- Figure 6 is a view analogous to those of Figures 3 to 5, after the addition of the other part of the powder intended to form the external layer 3,
- Figure 7 is a view analogous to those of Figures 3 and 6, during a final compression step,
- Figure 8 shows the average dissolution profiles of an uncoated tablet 1 according to the invention comprising a gastro-resistant layer 4b comprising 25%, 50% or 100% by weight of talc, relative to the weight of gastro-resistant polymer, Figure 8a showing the dissolution profiles of the external layer of clopidogrel powder in the first dissolution medium at a pH=1.2, Figure 8b showing the dissolution profiles of the aspirin core in the first dissolution medium at a pH of approximately or equal to 1.2, and Figure 8c showing the dissolution profiles of the aspirin core

in the second dissolution medium at a pH of approximately or equal to 6.8.

### Example 1: Tablet according to the invention

### a. Composition of the tablet 1 according to the invention

The percentages of the ingredients below are expressed, unless otherwise indicated, by dry weight relative to the total dry weight of the tablet which contains them, as appropriate.

### • Composition of the tablet 2 comprising aspirin

| **Ingredient** | **mg/tablet** |
|---|---|
| Starched aspirin | 111.111 |
| Stearic acid | 0.556 |
| Anhydrous colloidal silica | 0.333 |
| Microcrystalline cellulose | 11.666 |
| **Weight of aspirin core** | **123.666** |

### • Composition of the external layer 3 comprising clopidogrel

| **Ingredient** | **Brand** | **mg/tablet** |
|---|---|---|
| Clopidogrel hydrogen sulfate | | 97.880 |
| Anhydrous lactose | | 87.440 |
| Weakly substituted hydroxypropylcellulose | | 24.300 |
| PEG 6000 | Macrogol 6000P | 22.300 |
| Alpha-tocopherol | | 0.180 |
| Partially pregelatinised starch | | 14.500 |
| Microcrystalline cellulose | | 98.000 |
| Hydrogenated castor oil | | 3.300 |
| Fatty acid sucrose esters | | 2.100 |
| **Weight of external layer** | | **350.000** |

### • Composition of the three-layer coating 4

**- internal isolating layer 4a**

| **Ingredient** | **Brand** | **mg/tablet** |
|---|---|---|
| Hydroxypropylmethylcellulose | Hypromellose 6 mPa.s EP/USP/JP | 2.968 |
| PEG 6000 | Macrogol 6000P | 0.742 |
| **Weight of internal isolating layer** | | **3.710** |

**- gastro-resistant layer 4b**

| **Ingredient** | **Brand** | **mg/tablet** |
|---|---|---|
| Copolymer of methacrylic acid and of ethyl acrylate: | Eudragit L30D55 (expressed as amount of solids) | 17.982 |
| Talc | | 8.992 |
| Triethyl citrate | | 3.596 |
| **Weight of gastro-resistant layer** | | **30.570** |

**- external isolating layer 4c**

| **Ingredient** | **Brand** | **mg/tablet** |
|---|---|---|
| Hydroxypropylmethylcellulose | Hypromellose 6 mPa.s EP/USP/JP | 3.790 |
| PEG 6000 | Macrogol 6000P | 0.948 |
| **Weight of external isolating layer** | | **4.738** |

Weight of the uncoated tablet: 512.684 mg

### • Film-coating composition 5 (four layers)

| | **Ingredient** | **Brand** | **mg/tablet** |
|---|---|---|---|
| First layer | Hydroxypropylmethylcellulose | Hypromellose 6 mPa.s EP/USP/JP | 5.525 |
| | Talc | | 0.825 |
| Second layer | Titanium dioxide | | 4.735 |
| | PEG 6000 | Macrogol 6000P | 3.157 |
| | Hydroxypropylmethylcellulose | Hypromellose 6 mPa.s EP/USP/JP | 10.276 |
| | Talc | | 1.543 |
| | Mixture of polydimethylsiloxane and silica dioxide | PDMsiloxan SIL Dioxyde Mel | 0.039 |
| Third layer | Hydroxypropylmethylcellulose | Hypromellose 6 mPa.s EP/USP/JP | 0.740 |
| | PEG 6000 | Macrogol 6000P | 0.151 |
| | Mixture of polydimethylsiloxane and silica dioxide | PDMsiloxan SIL Dioxyde Mel | 0.002 |
| Fourth layer | Carnauba wax | | 0.003 |
| | Talc | | 0.004 |
| | **Weight of film coating** | | **27.000** |

Final weight of the film-coated tablet: 539.684 mg

### b. Structure of the tablet 1 according to the invention

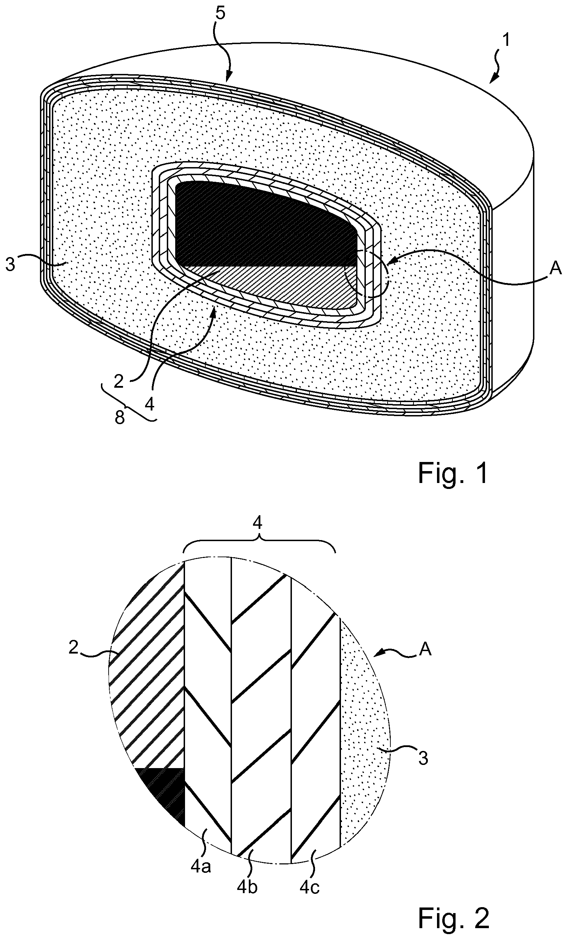

Represented in Figure 1 is a diagrammatic sectional view of a tablet 1 according to the invention. Usually, the tablet 1 has a cylindrical general shape and comprises two opposite, upper and lower, faces of convex shape.

The tablet 1 comprises a core 2 comprising aspirin, and an external layer 3 comprising clopidogrel hydrogen sulfate. The core 2 is preferably centred in the tablet 1 and has a general shape identical or similar to that of the tablet 1. In Figure 1, it therefore has a cylindrical general shape and comprises two opposite, upper and lower, faces of convex shape, like the tablet 1. In particular, the radius of curvature of each of the two convex opposite faces of the core will be identical or approximately identical to that of each of the two convex opposite faces of the tablet 1. The core 2 is separated from the external layer 3 by means of a three-layer coating 4.

The assembly composed of the core 2 and of the three-layer coating 4 forms the coated core 8. The three-layer coating 4 is more thoroughly detailed in Figure 2 described hereinafter.

Finally, the tablet 1 may comprise a film coating 5.

Without the film coating 5, the tablet 1 has a radius of approximately 5.55 mm and a maximum thickness of approximately 5.9 mm.

With the film coating 5, the tablet 1 has a radius of approximately 5.6 mm and a maximum thickness of approximately 6.0 mm.

According to one preferential embodiment, presented in Figure 1, the film coating 5 is composed of the following four layers, indicated from the inside to the outside of the tablet 1:
- a first attachment layer,
- a second layer, a function of which is to protect the clopidogrel from light,
- a third layer for obtaining a smooth surface appearance and which is capable of receiving optional impressions, and
- a fourth a layer aimed at promoting the sliding of the tablet during manipulation thereof (in particular, during the impression, packaging and ingestion operations).

Such a film coating is particularly suitable when it is desired to obtain a tablet with a pleasant aesthetic appearance.

Represented in Figure 2 is an enlarged view of the detail A of Figure 1 representing the three-layer coating 4 separating the core 2 from the external layer 3. The three-layer coating is composed of the following three layers, indicated from the inside to the outside of the tablet 1:
- an internal isolating layer, 4a,
- a gastro-resistant layer, 4b,
- an external isolating layer, 4c.

The internal isolating layer 4a and the external isolating layer 4c may have an identical or different chemical composition and/or thickness. Preferably, and as is indicated in Figure 2, the isolating layers 4a and 4c have a thickness which is smaller than that of the gastro-resistant layer 4b.

### c. General process for manufacturing the tablet according to the invention

For the whole of the description of the process for manufacturing the tablet according to the invention which is given hereinafter, the amounts of each of the ingredients are, unless otherwise indicated, those indicated in the composition tables given in point a) of this example. It will be noted that these amounts are expressed by weight (milligram) of each of the ingredients per tablet (mg/tab).

More particularly, the ingredients and the amounts indicated in point a) do not take into account the solvents (such as water or an alcohol) used for carrying out the process. Consequently, when the manufacturing process described hereinafter requires the use of a solvent, the amount of solvent is specifically specified, in mg/tab.

### Preparation of the powder intended to form the external layer 3 (clopidogrel powder)

The process for preparing the clopidogrel powder comprises a step of preparing clopidogrel granules, obtained by hot melt granulation.

The term "hot melt granulation" generally means the agglomeration of several ingredients, introduced in the form of particles, and some of which are hot-melt, in the form of granules. This agglomeration of the ingredients is carried out by mixing and heating at a temperature which allows the hot-melt ingredients to melt, and then the heated mixture of ingredients is cooled while being stirred, in order to obtain granules. If one (or more) of the ingredients subjected to the hot-melt granulation is (are) already in the form of granules, the size of the granules resulting from the hot-melt granulation will be greater than that of the granules of the initial ingredient(s).

The formation of clopidogrel granules is generally followed by mixing with several excipients so as to form the clopidogrel powder.

More particularly, the clopidogrel powder, the composition of which is described in point a) above, is prepared according to the following process:
The alpha-tocopherol and the pregelatinised starch are mixed. The clopidogrel, anhydrous lactose, hydroxypropylcellulose and macrogol are mixed and then added to and mixed with the mixture of alpha-tocopherol and the pregelatinised starch. The resulting mixture is then treated by hot-melt granulation in order to obtain, after cooling, the clopidogrel granules. The clopidogrel granules then preferably undergo a calibration step. The hydroxypropylcellulose and the microcrystalline cellulose are then added to and mixed with the (calibrated) clopidogrel granules. Finally, the hydrogenated castor oil and the sucrose ester are added to the mixture and mixed in order to form the clopidogrel powder.

### Preparation of the aspirin core 2

The core 2 comprising aspirin is prepared by direct compression of the ingredients of which it is composed.

More particularly, the aspirin core, the composition of which is described in point a) above, is prepared according to the following process:
The starched aspirin, the stearic acid, the anhydrous colloidal silica and the microcrystalline cellulose are mixed and then compressed using a rotary tableting machine equipped with dies 7 mm in diameter and with upper and lower punches 7 mm in diameter and with a radius of curvature of 10 mm.

### Three-layer coating 4 of the aspirin core 2

This step of the process comprises the successive deposition of the three layers of the three-layer coating 4 onto the aspirin core 2.

The three layers (internal isolating layer 4a, gastro-resistant layer 4b, external isolating layer 4c) are deposited by spraying an aqueous solution comprising the ingredients of each of the layers, each deposition being followed by drying.

More particularly, as regards the three-layer coating, the composition of which is described in point a) above, the various solutions are sprayed onto the loaded cores in a film-coating turbine using equipment comprising spray guns with nozzles that are 1.0 mm and 1.5 mm in diameter. The deposition, onto the cores, of the layers in film form and the drying are carried out using a hot air stream which passes through the body of the cores. This drying, concomitant with the spraying, makes it possible to deposit a dry film.

### • Deposition of the internal isolating layer 4a:

Pour the hydroxypropylmethylcellulose and the PEG 6000 into purified water (37.512 mg/tab) with moderate stirring and leave to stir until complete dissolution is obtained.

This solution is deposited by means of 1.0 mm nozzles and dried as indicated above.

### • Deposition of the gastro-resistant layer 4b:

Pour the talc into purified water (45.049 mg/tab) with vigorous stirring (stirrer) and maintain the stirring for 25 to 35 minutes.

Add the triethyl citrate with moderate stirring and maintain the stirring for 25 to 35 minutes.

Pour the dispersion of Eudragit L30D55, preferably while sieving it through a screen of approximately 0.8 mm, into the solution previously prepared and maintain slow stirring until the end of the film coating.

### Verify the absence of foam and of lumps.

This solution is deposited by means of nozzles having a diameter of 1.5 mm and dried as indicated above.

### • Deposition of the external isolating layer 4c:

This deposition is carried out in a manner similar to the deposition of the internal isolating layer 4a, but with an amount of purified water of 47.906 mg/tab.

### Preparation of the tablet 1

The tablet 1 is prepared by double compression of the coated aspirin core 8 inside the clopidogrel powder.

This double compression step is more particularly described in Figures 3 to 7 of the present application.

Represented in Figure 3 is a diagrammatic partial section of a compression unit of a tablet-producing machine. This compression unit comprises a die 6 comprising a circular wall. This compression unit has a vertical reference axis X'X which advantageously constitutes a central axis of symmetry, or even of rotation, for this compression unit. A lower punch 7 comprising a punch rod 7a is sunk in the die 6, the punch 7 being translationally mobile in the die 6, parallel to the axis X'X.

As is visible in Figure 3, the volume created by the die 6 and the punch 7 is filled with an amount slightly greater (approximately 5% to 10%) than half the total amount (185 mg) of the clopidogrel powder intended to form the external layer 3. At the end of the filling of this volume with the clopidogrel powder, the surface thereof is levelled.

Figure 4 shows a view analogous to that of Figure 3, after the punch 7 has been lowered into the die 6 via a translational movement along the axis X'X in order to slightly increase the volume created by the die 6 and the punch 7. The positioning of the coated core 8 is represented in this figure. Preferably, the coated core 8 is positioned on the reference axis X'X symmetrically. The presence, the integrity and the positioning of the coated core 8 is verified using a suitable device, such as a video camera.

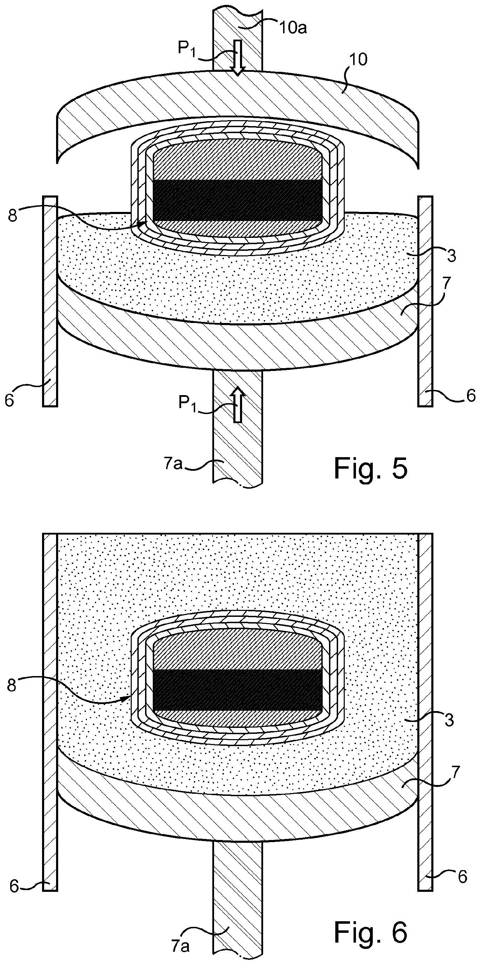

Figure 5 is a figure analogous to that of Figures 3 and 4, during an intermediate compression step. The other punch 10 bearing a rod 10a is represented in this figure. The reference axis X'X is also the common axis of the punches and, consequently, the pressure applied by the lower and upper punches is parallel to this direction, creating a symmetrical distribution of this pressure. The intermediate compression represented in figure 5 has the effect of very slightly pushing the coated core 8 into and positioning it in the part of clopidogrel powder intended to form the external layer 3, already present in the volume formed by the die 6 and the lower punch 7. At the end of this step, a pre-tablet is obtained.

Represented in Figure 6 is a view analogous to that of Figures 3 to 5, after the punch 7 has been lowered into the die 6 via a translational movement along the axis X'X in order to notably increase the volume created by the die 6 and the punch 7. In this figure, the remainder of the clopidogrel powder intended to form the external layer 3 has been added to the pre-tablet so as to fill the entire volume created by the die 6 and the punch 7. At the end of this addition of clopidogrel powder, the surface thereof is levelled.

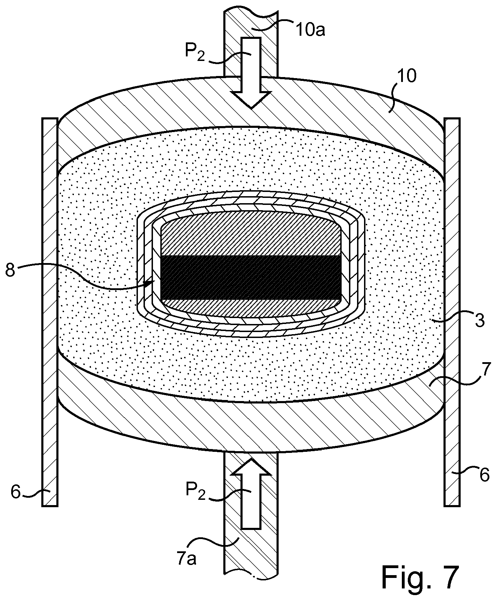

Finally, Figure 7 is a view analogous to that of Figures 3 to 6, during the final compression step. The pressure P2 applied during the step of this final compression is greater than the pressure P1 applied during the intermediate compression step represented in Figure 5.

By way of example, for the tablet of which the composition is described in point a) above, the double compression was carried out on a double-core rotary tableting machine equipped:
- with dies 11 mm in diameter and upper and lower punches 11 mm in diameter and with a radius of curvature of 10 mm,
- with the system for conveying and distributing the coated cores, and
- with the camera system for verifying and adjusting the positioning of the coated cores on the bed of clopidogrel powder intended to form the external layer.

The feeding with clopidogrel powder was carried out by gravitation loading of each of the feed hoppers of the tableting machine from the container of powder.

The feeding with coated aspirin cores was carried out by gravitation loading of the feed hopper of the core distributor from a stainless steel container.

The entire operation was carried out in premises maintained under controlled temperature and humidity conditions (RH ≤ 4.5 g of water/kg of dry air).

The tablet obtained at the end of this final compression is ejected. For example, the punch 7 slides up in the die 6 (a translational movement along the axis X'X), thus enabling the tablet to be ejected. Finally, the tablet can, subsequently, undergo one or more film coating steps.

### Film coating of the tablet

The tablet can be film-coated according to the usual film coating techniques. This involves successively depositing one or more layers on the tablet, the final layer possibly being a waxing of the tablet.

A film-coating layer is generally obtained by spray-coating with an aqueous-alcoholic solution comprising the ingredients of the layer, followed by drying.

More particularly, as regards the film coating of which the composition is described in point a) above, said coating comprises three layers deposited by spraying, the final layer been deposited by powdering.

The spraying operations can be carried out using the film-coating turbine equipped with the system for drying with a hot air stream, described above.

### • Deposition of the first layer:

Disperse the hydroxypropylmethylcellulose in ethanol (61.088 mg/tab) with stirring until complete solubilisation is obtained, then add the purified water (32.527 mg/tab) and the talc.

This solution is deposited by means of 1.5 mm nozzles and dried as indicated above.

### • Deposition of the second layer:

Pour the hydroxpropylmethylcelullose into ethanol (113.572 mg/tab), stir until complete solubilisation is obtained.

Then introduce the PEG 6000 and the mixture of polydimethylsiloxane and silica dioxide.

Mill the titanium dioxide in the purified water (56.655 mg/tab), then disperse the talc.

Pour this solution into the alcoholic solution.

This solution is deposited by means of nozzles having a diameter of 1.5 mm and dried as indicated above.

### • Deposition of the third layer:

Disperse the hydroxpropylmethylcelullose in ethanol (11.743 mg/tab) until complete solubilisation is obtained. Then add the mixture of polydimethylsiloxane and silica dioxide, the PEG 6000 and the purified water (6.24 mg/tab).

This solution is deposited by means of 1.5 mm nozzles and dried as indicated above.

### • Deposition of the fourth layer:

The carnauba wax and the talc are introduced by dusting onto the tablet mass kept rotating in the turbine.

### Example 2: Effect of the talc content

The effect of the talc content of the gastro-resistant layer on the gastroresistance of the aspirin contained in the core of a tablet according to the invention and on the dissolution profile of said tablet was more particularly studied.

### a. Materials and methods:

### 1. Obtaining the tablets

Tablets as described in Example 1 were prepared. The talc content of the gastro-resistant layer was varied in these tablets.

The tests were carried out on the following batches:
- batch of uncoated tablets having a gastro-resistant layer with a talc content of 25%,
- batch of uncoated tablets having a gastro-resistant layer with a talc content of 50%,
- batch of uncoated tablets having a gastro-resistant layer with a talc content of 100%,
the talc contents being given relative to the dry weight of gastro-resistant polymer (Eudragit L 30 D-55).

The tablets were prepared as in Example 1, with the exception of the film-coating step, which was omitted (tests carried out on uncoated tablets).

### 2. Compression

The compression material is that described in Example 1 c above.

On the basis of the compression parameters used for common productions, 3 series of compressive forces were studied:
- high-level forces of station 1 and 2 = R1,
- low-level forces of station 1 and 2 = R2,
- standard-level forces of station 1 and 2 = R3.

**Table 1: The various forces of station 1 and 2 tested for the double compression**

| | **Station 1** | **Station 2** |
|---|---|---|
| **High level (kN) = R1** | Approximately 2 | Approximately 25 |
| **Low level (kN) = R2** | Approximately 0.8 | Approximately 18 |
| **Standard level (kN) = R3** | Approximately 1.2 | Approximately 22 |

Station 1 applies the intermediate compressive force and station 2 applies the final compressive force.

Tables 2-4 hereinafter group together the parameters applied and the results of the testing obtained during the compression of the tablets.

### 3. Testing of the tablets

- Thickness, diameter: the diameter and the thickness of the tablets are tested using a comparator graduated in tenths of a millimetre.
- Uniformity of weight: the test for uniformity of weight is carried out according to European pharmacopoeia 2.9.5, using a balance, with a sensitivity of 0.1 mg, coupled to a printer. Twenty tablets are sampled and weighed individually. The average weight is determined, as is the coefficient of variation. For tablets having an average weight greater than 250 mg, the test is validated if a maximum of two results differ by +/-5% from the average value and none differ by +/-10%.

### 4. Crushing strength

The crushing strength test is carried out according to European pharmacopoeia 2.9.8, using a durometer. Ten tablets are sampled and placed one after another between the two jaws of the durometer. The value of the force exerted on the tablet, obtained during the crushing of the tablet, is noted. The test is validated if the individual values are greater than 4 kp.

### 5. Friability measurement

The friability measurement test is carried out according to European pharmacopoeia 2.9.7, on 6.5 g of tablets, i.e. approximately 13 tablets, for 4 min at 25 R/min. The percentage loss of weight is calculated and the absence or presence of a cleaved tablet is verified. The test is validated if the percentage loss of weight is less than or equal to 1% and no tablet is cleaved.

### 6. Disintegration test in medium with pH=1.2

The disintegration test in medium with pH 1.2 is carried out according to European pharmacopoeia 2.9.1, on 12 tablets with observation of the disintegration time for the external clopidogrel layer, and then after 120 minutes of test, the appearance of the cores is observed. The test is validated if the external layer is disintegrated in less than 15 min and if no core is disintegrated and/or no more than two cores are swollen.

### 7. Preliminary results

**Table 3: Test at 25% talc**

| **Characteristics parameters** | **R1** | | **R2** | | **R3** | |
|---|---|---|---|---|---|---|
| | **Station 1** | **Station 2** | **Station 1** | **Station 2** | **Station 1** | **Station 2** |
| **Machine rate (tabl/h)** | 50 000 | | 50 000 | | 50 000 | |
| **Shoe rotation speed (rpm)** | 35 | 45 | 35 | 45 | 35 | 45 |
| **Main compressive force (kN)** | 2.0 | 24.9 | 0.8 | 18.0 | 1.2 | 21.9 |
| **Filling height (mm)** | 2.53 | 6.05 | 2.52 | 6.82 | 2.52 | 6.53 |
| **Penetration (mm)** | 5.72 | 2.89 | 5.72 | 2.88 | 5.72 | 2.88 |
| **Weight (mg)** | 352.15 | 512.0 | 352.8 | 512.2 | 352.8 | 513.6 |
| **Centring** | compliant | | compliant | | compliant | |
| **Average hardness (kp)** | 8.8 | | 6.4 | | 7.2 | |
| **Min-max hardness (kp)** | 7.7 - 9.2 | | 5.2 - 8.5 | | 5.5 - 9.2 | |
| **Thickness (min-max)** | 5.74 - 5.81 | | 5.87 - 5.91 | | 5.76 - 5.83 | |
| **Friability (%) T4** | 0% | | 0% | | 0% | |
| **Disintegration of external layer at a pH=1.2 (min)** | 4 min | | 4 min | | 7 min | |
| **Disintegration of the core at a pH= 1.2** | After 120 min 12 non-compliant disintegrated tablets | | After 120 min 12 non-compliant disintegrated tablets | | After 120 min 10 non-compliant disintegrated tablets | |

**Table 4: Test at 50% talc**

| **Characteristics parameters** | **R1** | | **R2** | | **R3** | |
|---|---|---|---|---|---|---|
| | **Station 1** | **Station 2** | **Station 1** | **Station 2** | **Station 1** | **Station 2** |
| **Machine rate (tabl/h)** | 50 000 | | 50 000 | | 50 000 | |
| **Shoe rotation speed (rpm)** | 35 | 45 | 35 | 45 | 35 | 45 |
| **Main compressive force (kN)** | 1.9 | 25.4 | 0.8 | 18.5 | 1.2 | 21.7 |
| **Filling height (mm)** | 2.56 | 6.04 | 2.56 | 6.77 | 2.56 | 6.51 |
| **Penetration (mm)** | 5.72 | 2.89 | 5.72 | 2.89 | 5.72 | 2.89 |
| **Weight (mg)** | 349.9 | 512.5 | 350.2 | 516.9 | 349.85 | 515.75 |
| **Centring** | compliant | | compliant | | compliant | |
| **Average hardness (kp)** | 8.5 | | 6.7 | | 7.4 | |
| **Min-max hardness (kp)** | 6.6 - 10.4 | | 5.3 - 9.4 | | 5.2 - 9.2 | |
| **Thickness (min-max)** | 5.72 - 5.78 | | 5.82 - 5.86 | | 5.79 - 5.80 | |
| **Friability (%) T4** | 0% | | 0% | | 0% | |
| **Disintegration of external layer at a pH=1.2 (min)** | 8 min | | 6 min | | 7 min | |
| **Disintegration of the core at a pH= 1.2** | After 120 min 2 tablets swollen but not disintegrated, therefore compliant | | Compliant, interruption after 120 min | | Compliant, interruption after 120 min | |

**Table 5: Test at 100% talc**

| **Characteristics parameters** | **R1** | | **R2** | | **R3** | |
|---|---|---|---|---|---|---|
| | **Station 1** | **Station 2** | **Station 1** | **Station 2** | **Station 1** | **Station 2** |
| **Machine rate (tabl/h)** | 50 000 | | 50 000 | | 50 000 | |
| **Shoe rotation speed (rpm)** | 35 | 45 | 35 | 45 | 35 | 45 |
| **Main compressive force (kN)** | 2.0 | 24.4 | 0.9 | 18.0 | 1.2 | 22.0 |
| **Filling height (mm)** | 2.49 | 6.04 | 2.52 | 6.90 | 2.51 | 6.54 |
| **Penetration (mm)** | 5.72 | 2.89 | 5.72 | 2.88 | 5.72 | 2.88 |
| **Weight (mg)** | 351.0 | 512.7 | 350.8 | 516.1 | 350.5 | 515.7 |
| **Centring** | compliant | | compliant | | compliant | |
| **Average hardness (kp)** | 8.7 | | 7.1 | | 6.1 | |
| **Min-max hardness (kp)** | 7.6 - 10.8 | | 5.0 - 9.0 | | 4.6-8.1 | |
| **Thickness (min-max)** | 5.73-5.74 | | 5.77 - 5.86 | | 5.7-5.8 | |
| **Friability (%) T4** | 0% | | 0% | | 0% | |
| **Disintegration of external layer at a pH=1.2 (min)** | 5 min | | 5 min | | 7 min | |
| **Disintegration of the core at a pH=1.2** | Compliant, interruption after 120 min | | Compliant, interruption after 120 min | | Compliant, interruption after 120 min | |

In terms of physical characteristics (weight, hardness, thickness, friability, disintegration time of the external layer), the talc percentage of the gastro-resistant layer has only a slight effect. On the other hand, this talc percentage has a notable influence on the gastroresistance of the aspirin core. In particular, it is confirmed that an aspirin core of which the gastro-resistant layer contains 25% of talc does not withstand the double compression phase and results in aspirin cores which are not gastro-resistant.

### 8. Establishment of dissolution profiles

The establishment of the dissolution profile is carried out according to Japanese pharmacopoeia 6.10, on six tablets. The assaying of the clopidogrel and of the aspirin is carried out by liquid phase chromatography, according to Japanese pharmacopoeia 2.01.

The tablets are immersed in a first acidic dissolution medium at a pH of approximately or equal to 1.2. Samples are taken at 15 and 30 minutes and the clopidogrel is assayed. After two hours in this first dissolution medium, the aspirin is assayed. 10% of the total dose of aspirin must at most be found in the medium. Next, the tablets are immersed individually in a second dissolution medium at a pH of approximately or equal to 6.8. Samples are taken at 30, 40, 50, 60, 75, 90, 105 and 120 minutes, and the aspirin is assayed.

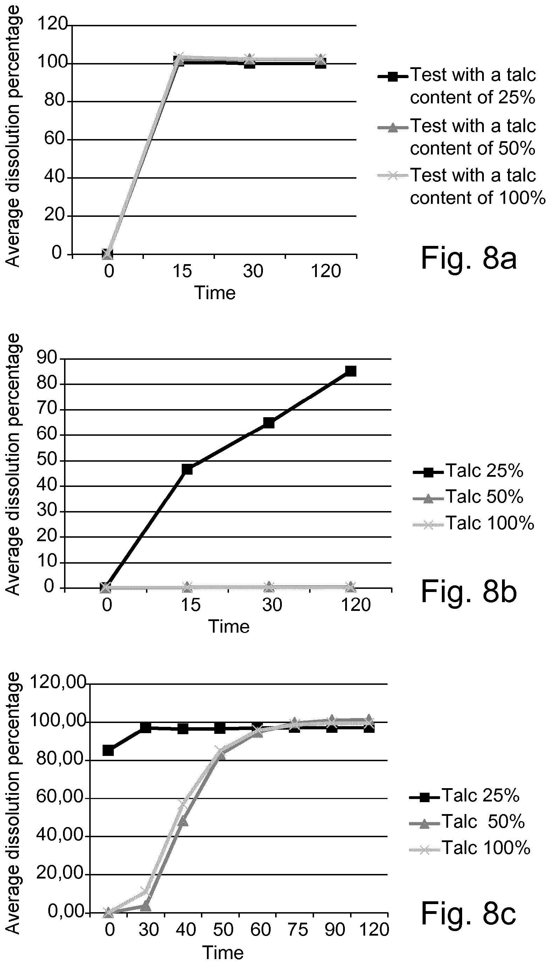

The average dissolution profiles are indicated in Figure 8 for uncoated tablets comprising 25%, 50% or 100% by weight of talc in the gastro-resistant layer. Figure 8a shows the dissolution profiles of the external layer of clopidogrel powder in the first dissolution medium, Figure 8b shows the dissolution profiles of the aspirin core in the first dissolution medium, and Figure 8c shows the dissolution profiles of the aspirin core in the second dissolution medium.

It is noted in these figures that a gastro-resistant layer containing 25% of talc does not make it possible to obtain a tablet of which the aspirin core is gastro-resistant.

### Example 3: Stability

The conditions for storing a tablet according to the invention and a tablet according to application WO 00/66130, packaged in an aluminium blister pack sealed with a sheet of aluminium, are compared in the table below:

| | |
|---|---|
| Tablet according to WO 00/66130 | • Storage time of 24 months, "store at a temperature below 25°C" |
| | • Storage time of 18 months, "store at a temperature below 30°C" |
| Tablet according to the invention | • Storage time of 36 months. No particular storage conditions. |

These storage conditions clearly illustrate the better stability of the tablet according to the invention. These storage conditions are therefore less restrictive than those of the tablet according to WO 00/66130 and they prove to be particularly advantageous for countries which can have temperatures above 25 or 30°C.

## Claims

1. Pharmaceutical tablet comprising a core and an external layer, the core comprising acetylsalicylic acid or a pharmaceutically acceptable salt thereof and the external layer comprising clopidogrel or a pharmaceutically acceptable salt thereof, the core being separated from the external layer by a three-layer coating composed of a gastro-resistant layer inserted between two isolating layers, the gastro-resistant layer comprising at least one gastro-resistant polymer and at least 40% by weight of talc, relative to the weight of gastro-resistant polymer.

2. Tablet according to Claim 1, the tablet of which has a volume of less than 500 mm³.

3. Tablet according to either one of the preceding claims, such that the tablet is capable of releasing in vitro:
- at least 70% by weight of clopidogrel or the pharmaceutically acceptable salt thereof in a first acidic dissolution medium at a pH of approximately or equal to 1.2 with a type 1 dissolution apparatus in accordance with Japanese pharmacopoeia 6.10 "Dissolution test", over the course of 30 min while at the same time releasing at most 10% by weight of acetylsalicylic acid or the pharmaceutically acceptable salt thereof, over the course of 120 min, then
- at least 65% by weight of acetylsalicylic acid or the pharmaceutically acceptable salt thereof in a second dissolution medium of buffered solution at a pH of approximately or equal to 6.8 with a type 1 dissolution apparatus in accordance with Japanese pharmacopoeia 6.10 "Dissolution test", over the course of 90 min.

4. Tablet according to any one of the preceding claims, in which the composition of the isolating players is identical and comprises hydroxypropylmethylcellulose (HPMC), hydroxypropylcellulose and/or polyvinyl alcohol.

5. Tablet according to any one of the preceding claims, in which the clopidogrel and the acetylsalicylic acid are present in a clopidogrel/acetylsalicylic acid molar ratio of between 0.35 and 0.45.

6. Process for manufacturing a tablet according to any one of Claims 1 to 5, said process comprising the following steps:
(i) preparing a core comprising acetylsalicylic acid or a pharmaceutically acceptable salt thereof,
(ii) coating the core, so as to obtain a coated core, with a three-layer coating composed of a gastro-resistant layer inserted between two isolating layers,
(iii) compression-coating the coated core obtained in step (ii) with the powder comprising the clopidogrel or a pharmaceutically acceptable salt thereof, so as to obtain a tablet.

7. Process according to Claim 6, in which step (ii) is carried out by means of three successive coating steps.

8. Process according to Claim 7, in which the core bearing a first isolating layer is coated with an amount of gastro-resistant layer representing at least 20% by weight, relative to the total weight of the core.

9. Process according to any one of Claims 6 to 8, in which the ratio of the weight of the external layer to the weight of the core is included in a range from 2.5 to 3.2.

10. Process according to any one of Claims 6 to 9, in which step (iii) is a double compression carried out in two substeps:
- an intermediate compression of the core coated with a first part of the clopidogrel powder in order to obtain a pre-tablet, then
- a final compression of the pre-tablet with the other part of the clopidogrel powder.

11. Process according to Claim 10, in which the compressive force applied during the intermediate compression is less than the compressive force applied during the final compression.

12. Process according to any one of preceding Claims 6 to 11, also comprising a film-coating step.

13. Tablet according to any one of Claims 1 to 5, for use in the treatment of a pathological condition induced by platelet aggregation, including stable or unstable angina, cardiovascular and cerebrovascular system disorders, for instance thromboembolic disorders associated with atherosclerosis and with diabetes, such as unstable angina, stroke, post-angioplasty restenosis, endarterectomy or the insertion of metal endovascular prostheses, or thromboembolic disorders associated with the post-thrombolysis re-thrombosis, with infarction, with dementia of ischaemic origin, with peripheral arterial diseases, with haemodialysis, with atrial fibrillation, or else during the use of vascular prostheses, of aortocoronary bypasses, or else during radiotherapy in order to reduce the side effects thereof.

## Patentansprüche

1. Pharmazeutische Tablette, umfassend einen Kern und eine äußere Schicht, wobei der Kern Acetylsalicylsäure oder ein pharmazeutisch verträgliches Salz davon umfasst und die äußere Schicht Clopidogrel oder ein pharmazeutisch verträgliches Salz davon umfasst, wobei der Kern von der äußeren Schicht durch eine dreischichtige Beschichtung getrennt ist, die aus einer magensaftresistenten Schicht, die zwischen zwei isolierende Schichten eingefügt ist, besteht, wobei die magensaftresistente Schicht wenigstens ein magensaftresistentes Polymer und wenigstens 40 Gew.-% Talkum, bezogen auf das Gewicht des magensaftresistenten Polymers, umfasst.

2. Tablette gemäß Anspruch 1, wobei die Tablette ein Volumen von weniger als 500 mm³ aufweist.

3. Tablette gemäß einem der vorstehenden Ansprüche, wobei die Tablette fähig ist, *in vitro* freizusetzen:
- wenigstens 70 Gew.-% des Clopidogrels oder des pharmazeutisch verträglichen Salzes davon in einem ersten sauren Auflösungsmedium mit einem pH-Wert von etwa oder gleich 1,2 in einer Typ-1-Auflösungsvorrichtung gemäß dem Japanischen Arzneibuch 6.10, "Auflösungsprüfung", im Verlauf von 30 min, während zugleich höchstens 10 Gew.-% der Acetylsalicylsäure oder des pharmazeutisch verträglichen Salzes davon im Verlauf von 120 min freigesetzt wird, dann
- wenigstens 65 Gew.-% der Acetylsalicylsäure oder des pharmazeutisch verträglichen Salzes davon in einem zweiten Auflösungsmedium mit einer gepufferten Lösung mit einem pH-Wert von etwa oder gleich 6,8 in einer Typ-1-Auflösungsvorrichtung gemäß den Japanischen Arzneibuch 6.10, "Auflösungsprüfung", im Verlauf von 90 min.

4. Tablette gemäß einem der vorstehenden Ansprüche, wobei die Zusammensetzungen der isolierenden Schichten gleich sind und Hydroxypropylmethylcellulose (HPMC), Hydroxypropylcellulose und/oder Polyvinylalkohol umfassen.

5. Tablette gemäß einem der vorstehenden Ansprüche, wobei das Clopidogrel und die Acetylsalicylsäure in einem Clopidogrel/Acetylsalicylsäure-Molverhältnis von zwischen 0,35 und 0,45 vorhanden sind.

6. Verfahren zum Herstellen einer Tablette gemäß einem der Ansprüche 1 bis 5, wobei das Verfahren folgende Schritte umfasst:
(i) Herstellen eines Kerns, der Acetylsalicylsäure oder ein pharmazeutisch verträgliches Salz davon umfasst,
(ii) Beschichten des Kerns mit einer dreischichtigen Beschichtung, die aus einer magensaftresistenten Schicht, die zwischen zwei isolierende Schichten eingefügt ist, besteht, um einen beschichteten Kern zu erhalten,
(iii) Kompressionsbeschichten des bei Schritt (ii) erhaltenen beschichteten Kerns mit dem Pulver, das das Clopidogrel oder ein pharmazeutisch verträgliches Salz davon umfasst, um eine Tablette zu erhalten.

7. Verfahren gemäß Anspruch 6, wobei Schritt (ii) mithilfe von drei aufeinanderfolgenden Beschichtungsschritten durchgeführt wird.

8. Verfahren gemäß Anspruch 7, wobei der Kern, der eine erste isolierende Schicht trägt, mit einer Menge an magensaftresistenter Schicht beschichtet wird, die wenigstens 20 Gew.-%, bezogen auf das Gesamtgewicht des Kerns, darstellt.

9. Verfahren gemäß einem der Ansprüche 6 bis 8, wobei das Verhältnis des Gewichts der äußeren Schicht zu dem Gewicht des Kerns in einem Bereich von 2,5 bis 3,2 liegt.

10. Verfahren gemäß einem der Ansprüche 6 bis 9, wobei Schritt (iii) eine Doppelkompression ist, die in zwei Teilschritten durchgeführt wird:
- eine Zwischenkompression des Kerns, der mit einem ersten Teil des Clopidogrel-Pulvers beschichtet ist, um eine Vortablette zu erhalten, dann
- eine Endkompression der Vortablette mit dem anderen Teil des Clopidogrel-Pulvers.

11. Verfahren gemäß Anspruch 10, wobei die Kompressionskraft, die während der Zwischenkompression angelegt wird, kleiner als die während der Endkompression angelegte Kompressionskraft ist.

12. Verfahren gemäß einem der vorstehenden Ansprüche 6 bis 11, ferner umfassend einen Filmbeschichtungsschritt.

13. Tablette gemäß einem der Ansprüche 1 bis 5 für die Verwendung bei der Behandlung eines von Plättchenaggregation induzierten pathologischen Zustands, einschließlich stabiler oder instabiler Angina, Störungen des kardiovaskulären und zerebrovaskulären Systems, beispielsweise thromboembolische Störungen in Verbindung mit Atherosklerose und mit Diabetes, wie z. B. instabile Angina, Schlaganfall, Post-Angioplastik-Restenose, Endarterektomie oder Einführen von metallischen endovaskulären Prothesen, oder thromboembolische Störungen in Verbindung mit Post-Thrombolyse-Rethrombose, mit Infarkt, mit Demenz mit ischämischem Ursprung, mit peripheren Arterienerkrankungen, mit Hämodialyse, mit Vorhofflimmern oder bei der Verwendung von Gefäßprothesen, von Aortokoronar-Bypassen oder bei Strahlentherapie zum Verringern der Nebenwirkungen davon.

## Revendications

1. Comprimé pharmaceutique comprenant un noyau et une couche externe, le noyau comprenant de l'acide acétylsalicylique ou l'un des sels pharmaceutiquement acceptables de celui-ci et la couche externe comprenant du clopidogrel ou l'un des sels pharmaceutiquement acceptables de celui-ci, le noyau étant séparé de la couche externe par un enrobage à trois couches composées d'une couche gastrorésistante insérée entre deux couches isolantes, la couche gastrorésistante comprenant au moins un polymère gastrorésistant et au moins 40 % en masse de talc, par rapport à la masse du polymère gastrorésistant.

2. Comprimé selon la Revendication 1, ledit comprimé présentant un volume de moins de 500 mm³.

3. Comprimé selon l'une quelconque des revendications précédentes, de sorte que le comprimé soit capable de libérer *in vitro* :
- au moins 70 % en masse de clopidogrel ou du sel pharmaceutiquement acceptable de celui-ci dans un premier milieu de dissolution acide à un pH égal ou environ égal à 1,2 avec un dispositif de dissolution de type 1 conforme à l'essai de dissolution 6.10 de la pharmacopée japonaise, sur une durée de 30 minutes, tout en libérant au même moment un maximum de 10 % en masse d'acide acétylsalicylique ou du sel pharmaceutiquement acceptable de celui-ci, sur une durée de 120 minutes, puis
- au moins 65 % en masse d'acide acétylsalicylique ou du sel pharmaceutiquement acceptable de celui-ci dans un deuxième milieu de dissolution de solution tampon à un pH égal ou environ égal à 6,8 avec un dispositif de dissolution de type 1 conforme à l'essai de dissolution 6.10 de la pharmacopée japonaise, sur une durée de 90 minutes.

4. Comprimé selon l'une quelconque des revendications précédentes, dans lequel la composition des couches isolées est identique et comprend de l'hydroxypropylméthylcellulose (HPMC), de l'hydroxypropylcellulose et/ou de l'alcool polyvinylique.

5. Comprimé selon l'une quelconque des revendications précédentes, dans lequel le clopidogrel et l'acide acétylsalicylique sont présents dans un rapport molaire clopidogrel/acide acétylsalicylique compris entre 0,35 et 0,45.

6. Procédé de fabrication d'un comprimé selon l'une quelconque des Revendications 1 à 5, ledit procédé comprenant les étapes suivantes :
(i) préparation d'un noyau comprenant de l'acide acétylsalicylique ou l'un des sels pharmaceutiquement acceptables de celui-ci,
(ii) enrobage du noyau de sorte à obtenir un noyau enrobé avec un enrobage à trois couches composé d'une couche gastrorésistante insérée entre deux couches isolantes,
(iii) enrobage par compression du noyau enrobé obtenu dans l'étape (ii) avec la poudre comprenant le clopidogrel ou l'un des sels pharmaceutiquement acceptables de celui-ci, de sorte à obtenir un comprimé.

7. Procédé selon la Revendication 6, dans lequel l'étape (ii) est mise en oeuvre par le biais de trois étapes de revêtements successives.

8. Procédé selon la Revendication 7, dans lequel le noyau portant une première couche isolante est enrobé par une quantité de couche gastrorésistante représentant au moins 20 % en masse, par rapport à la masse totale du noyau.

9. Procédé selon l'une quelconque des Revendications 6 à 8, dans lequel le rapport de la masse de la couche externe sur la masse du noyau est inclus dans un intervalle allant de 2,5 à 3,2.

10. Procédé selon l'une quelconque des Revendications 6 à 9, dans lequel l'étape (iii) est une double compression mise en oeuvre en deux sous-étapes :
- une compression intermédiaire du noyau enrobé d'une première partie de la poudre de clopidogrel pour obtenir un pré-comprimé, puis
- une compression finale du pré-comprimé avec l'autre partie de la poudre de clopidogrel.

11. Procédé selon la Revendication 10, dans lequel la force de compression appliquée pendant la compression intermédiaire est inférieure à la force de compression appliquée pendant la compression finale.

12. Procédé selon l'une quelconque des Revendications 6 à 11 précédentes, comprenant également une étape d'enrobage par une pellicule.

13. Comprimé selon l'une quelconque des Revendications 1 à 5, pour utilisation dans le traitement d'un état pathologique induit par : agrégation plaquettaire, y compris angine instable et instable, trouble du système cardio-vasculaire et cérébro-vasculaire, par exemple troubles thrombo-emboliques associés à une athérosclérose et au diabète, par exemple angine instable, accident vasculaire cérébral, resténose post-angioplastie, endartériectomie ou insertion de prothèses endovasculaires métalliques, ou troubles thrombo-emboliques associés à la thrombose post-thrombolyse, avec infarctus, avec démence d'origine ischémique, avec maladies artérielles périphériques, avec hémodialyse, avec fibrillation auriculaire, ou encore pendant l'utilisation de prothèses vasculaires, de pontages aortocoronariens, ou encore pendant une radiothérapie pour réduire ses effets secondaires.
